# EUROPEAN PATENT APPLICATION

(11) **EP 1 356 764 A2**
(43) Date of publication of application: **29.10.2003**
(21) Application number: 03005269.0
(22) Date of filing: 10.03.2003
(51) Int. Cl.: A61B 5/02, A61B 5/00

(54) **Living subject monitoring apparatus**

(30) Priority: 24.04.2002 JP 2002123065
(71) Applicant: Colin Corporation, Komaki-shi, Aichi-ken (JP)
(72) Inventor: Nunome, Tomohiro, Komaki-shi, Aichi-ken (JP)
(74) Representative: Winter, Brandl, Fürniss, Hübner, Röss, Kaiser, Polte Partnerschaft

(57) **Abstract**

A living-subject monitoring apparatus (8) for monitoring a load exerted on a living subject, comprising a circulatory-organ-related-parameter measuring device (10,14,22,24,80,104,106,108) which iteratively measures at least one circulatory-organ-related parameter of the subject that changes in relation with the load exerted on the subject; a blood-oxygen-saturation measuring device (38,100,102) which iteratively measures a blood oxygen saturation of the subject; and a display device (32,114) which simultaneously displays the circulatory-organ-related parameter measured by the circulatory-organ-related-parameter measuring device and the blood oxygen saturation measured by the blood-oxygen-saturation measuring device, so that the circulatory-organ-related parameter and the blood oxygen saturation are comparable with each other by a medical person or the subject.

## Description

### BACKGROUND OF THE INVENTION

### Field of the Invention

The present invention relates to a living-subject monitoring apparatus for monitoring the circulatory organ of a living subject under load.

### Related Art Statement

There is known a living-subject monitoring apparatus that monitors the circulatory organ of a living subject under load, by displaying a timewise trend of at least one of blood pressure, heart rate, and product of blood pressure and heart rate of the subject each of which changes in relation with the load exerted to the subject, or producing an alarm when said at least one parameter does not fall in a normal range. The living-subject monitoring apparatus can advantageously monitor a living subject under load, such as a subject who is undergoing physical exercise or has undergone a surgical operation.

However, for example, in the case where a living subject who is undergoing an exercise test has an abnormality with the respiratory organ and accordingly cannot sufficiently exchange gases, the subject must stop the exercise test immediately. However, the above-indicated conventional apparatus that monitors the subject with respect to the blood pressure, the heart rate, or the product of blood pressure and heart rate, has a problem that it may not quickly recognize that the subject must stop the exercise test.

### SUMMARY OF THE INVENTION

It is therefore an object of the present invention to provide a living-subject monitoring apparatus that can quickly find that the ventilating ability of lungs of a living subject is abnormally or insufficiently low.

The above object has been achieved by the present invention. According to a first aspect of the present invention, there is provided a living-subject monitoring apparatus for monitoring a load exerted to a living subject, comprising a circulatory-organ-related-parameter measuring device which iteratively measures at least one circulatory-organ-related parameter of the subject that changes in relation with the load exerted to the subject; a blood-oxygen-saturation measuring device which iteratively measures a blood oxygen saturation of the subject; and a display device which simultaneously displays the circulatory-organ-related parameter measured by the circulatory-organ-related-parameter measuring device and the blood oxygen saturation measured by the blood-oxygen-saturation measuring device, so that the circulatory-organ-related parameter and the blood oxygen saturation are comparable with each other.

According to this aspect, the display device displays the circulatory-organ-related parameter that changes in relation with the load exerted to the subject, and the blood oxygen saturation that represents a degree of saturation of oxygen in blood of the subject, such that the circulatory-organ-related parameter and the blood oxygen saturation are comparable with each other by a living person such as a medical person or the subject. Therefore, based on an increasing tendency of the circulatory-organ-related parameter and a decreasing tendency of the blood oxygen saturation, the person can quickly recognize that the ventilating ability of the lungs of the subject whose circulatory organ is undergoing the load is abnormally or insufficiently low.

Preferably, the at least one circulatory-organ-related parameter measured by the circulatory-organ-related-parameter measuring device comprises at least one of a blood pressure, a heart rate, and a product of a blood pressure and a heart rate, of the subject. According to this feature, the display device displays at least one of the blood pressure, the heart rate, and the product of blood pressure and heart rate, and the blood oxygen saturation, such that the one parameter and the blood oxygen saturation are comparable with each other by a living person. Therefore, based on an increasing tendency of the one parameter and a decreasing tendency of the blood oxygen saturation, the person can quickly recognize that the ventilating ability of the lungs of the subject whose circulatory organ is undergoing the load is abnormally or insufficiently low.

Also, preferably, the display device simultaneously displays respective values of the circulatory-organ-related parameter that are iteratively measured by the circulatory-organ-related-parameter measuring device, and respective values of the blood oxygen saturation that are iteratively measured by the blood-oxygen-saturation measuring device, so that the respective values of the circulatory-organ-related parameter and the respective values of the blood oxygen saturation are comparable with each other. According to this feature, an increasing tendency of the circulatory-organ-related parameter, such as the blood pressure, the heart rate, and the product of blood pressure and heart rate, and a decreasing tendency of the blood oxygen saturation, can be easily recognized.

According to a second aspect of the present invention, there is provided a living-subject monitoring apparatus for monitoring a load exerted to a living subject, comprising a blood-oxygen-saturation measuring device which iteratively measure a blood oxygen saturation of the subject; a volumetric-pulse-wave detecting device which iteratively detects a volumetric pulse wave from the subject; and a supplied-oxygen-amount-index calculating device which iteratively calculates, based on a product of the blood oxygen saturation measured by the blood-oxygen-saturation measuring device and a volumetric-pulse-wave-related parameter related to the volumetric pulse wave detected by the volumetric-pulse-wave detecting device, a supplied-oxygen-amount index indicative of an amount of oxygen supplied to the subject.

According to this aspect, the supplied-oxygen-amount-index calculating device iteratively calculates, based on the product of the blood oxygen saturation and the volumetric-pulse-wave-related parameter (e.g., amplitude or area) related to the volumetric pulse wave detected by the volumetric-pulse-wave detecting device, the supplied-oxygen-amount index indicative of the amount of oxygen supplied to the subject. Based on the supplied-oxygen-amount index, it is possible to quickly recognize or detect that the ventilating ability of the lungs of the subject whose circulatory organ is undergoing the load is abnormally or insufficiently low.

Also, preferably, the living-subject monitoring apparatus further comprises a circulatory-organ-related-parameter measuring device which iteratively measures at least one circulatory-organ-related parameter of the subject that changes in relation with the load exerted to the subject; and a display device which simultaneously displays respective values of the circulatory-organ-related parameter that are iteratively measured by the circulatory-organ-related-parameter measuring device, and respective values of the supplied-oxygen-amount index that are iteratively calculated by the supplied-oxygen-amount-index calculating device, so that the respective values of the circulatory-organ-related parameter and the respective values of the supplied-oxygen-amount index are comparable with each other. According to this feature, the display device displays the respective values of the circulatory-organ-related parameter and the respective values of the supplied-oxygen-amount index, such that the respective values of the circulatory-organ-related parameter and the respective values of the supplied-oxygen-amount index are comparable with each other by a living person. Based on an increasing tendency of the circulatory-organ-related parameter and a decreasing tendency of the supplied-oxygen-amount index, the person can quickly recognize that the ventilating ability of the lungs of the subject whose circulatory organ is undergoing the load is abnormally or insufficiently low.

According to a third aspect of the present invention, there is provided a living-subject monitoring apparatus for monitoring a load exerted to a living subject, comprising: a circulatory-organ-related-parameter measuring device which iteratively measures at least one circulatory-organ-related parameter of the subject that changes in relation with the load exerted to the subject; a blood-oxygen-saturation measuring device which iteratively measures a blood oxygen saturation of the subject; and a load-index calculating device which iteratively calculates, based on a value obtained by dividing the circulatory-organ-related parameter measured by the blood-oxygen-saturation measuring device, by the blood oxygen saturation measured by the blood-oxygen-saturation measuring device, a load index indicative of the load exerted to the subject.

According to this aspect, the load-index calculating device iteratively calculates, based on the value obtained by dividing the circulatory-organ-related parameter measured by the blood-oxygen-saturation measuring device, by the blood oxygen saturation measured by the blood-oxygen-saturation measuring device, the load index indicative of the load exerted to the subject. Based on the load index, it is possible to quickly recognize or detect that the ventilating ability of the lungs of the subject whose circulatory organ is undergoing the load is abnormally or insufficiently low.

Also, preferably, the living-subject monitoring apparatus further comprises a display device which simultaneously displays respective values of the circulatory-organ-related parameter measured by the circulatory-organ-related-parameter measuring device, and respective values of the load index calculated by the load-index calculating device, so that the respective values of the circulatory-organ-related parameter and the respective values of the load index are comparable with each other. According to this feature, a time-wise relative change between an increasing tendency of the circulatory-organ-related parameter, such as blood pressure, heart rate, and product of blood pressure and heart rate, and an increasing tendency of the load index, can be easily recognized.

Preferably, the at least one circulatory-organ-related parameter measured by the circulatory-organ-related-parameter measuring device comprises a product of a blood pressure and a heart rate of the subject, and the load-index calculating device calculates, based on a value obtained by dividing the product of blood pressure and heart rate by the blood oxygen saturation measured by the blood-oxygen-saturation measuring device, the load index indicative of the load exerted to the subject. According to this feature, the load-index calculating device calculates, based on the value obtained by dividing the product of blood pressure and heart rate by the blood oxygen saturation, the load index indicative of the load exerted to the subject. Thus, based on the load index that reflects the respective changes of the load and the oxygen saturation, it is possible to quickly recognize that the ventilating ability of the lungs of the subject whose circulatory organ is undergoing the load is abnormally or insufficiently low.

### BRIEF DESCRIPTION OF THE DRAWINGS

The above and optional objects, features, and advantages of the present invention will be better understood by reading the following detailed description of the preferred embodiments of the invention when considered in conjunction with the accompanying drawings, in which:
Fig. 1 is a diagrammatic view for explaining a construction of a living-subject monitoring apparatus to which the present invention is applied;
Fig. 2 is a view for explaining a construction of a pressure-pulse-wave sensor which is employed by the monitoring apparatus of Fig. 1 so as to detect a pressure pulse wave from a living subject;
Fig. 3 is a block diagram for explaining essential control functions of an electronic control device shown in Fig. 1;
Fig. 4 is a flow chart for explaining the essential control functions of the electronic control device shown in Fig. 1;
Fig. 5 is a graph showing an example of physical information displayed by a display device of the monitoring apparatus of Fig. 1.

### DETAILED DESCRIPTION OF PREFERRED EMBODIMENTS

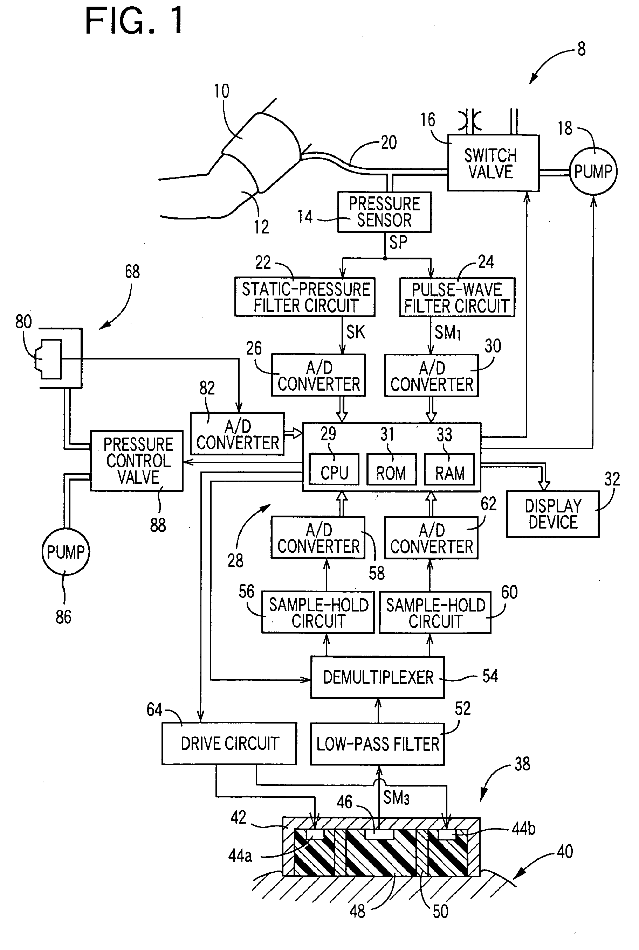

Hereinafter, there will be described an embodiment of the present invention in detail by reference to the accompanying drawings. Fig. 1 shows a view for explaining a construction of a living-subject monitoring apparatus 8 to which the present invention is applied. The present monitoring apparatus has a blood-pressure measuring function, a heart-rate measuring function, an oxygen-saturation measuring function, a blood-pressure-heart-rate-product measuring function, a supplied-oxygen-amount measuring function, and a load-index measuring function.

In Fig. 1, the living-subject monitoring apparatus 8 includes a cuff 10 which has a belt-like cloth bag and a rubber bag accommodated in the cloth bag and which is adapted to be wound around, e.g., an upper arm 12 of a patient as a living subject, and a pressure sensor 14, a switch valve 16, and an air pump 18 each of which is connected to the cuff 10 via a piping 20. The switch valve 16 is selectively placed in an inflation position in which the switch valve 16 permits a pressurized air to be supplied from the air pump 18 to the cuff 10, a slow-deflation position in which the switch valve 16 permits the pressurized air to be slowly discharged from the cuff 10, and a quick-deflation position in which the switch valve 16 permits the pressurized air to be quickly discharged from the cuff 10.

The pressure sensor 14 detects an air pressure in the cuff 10, and supplies a pressure signal SP representing the detected pressure, to each of a static-pressure filter circuit 22 and a pulse-wave filter circuit 24. The static-pressure filter circuit 22 includes a low-pass filter and extracts, from the pressure signal SP, a static-pressure component contained in the signal SP, i.e., a cuff-pressure signal SK representing the static pressure in the cuff 10. The cuff-pressure signal SK is supplied to an electronic control device 28 via an A/D (analog-to-digital) converter 26. The pulse-wave filter circuit 24 includes a band-pass filter and extracts, from the pressure signal SP, an oscillating component having predetermined frequencies, i.e., a pulse-wave signal SM₁. The pulse-wave signal SM₁ is supplied to the control device 28 via an A/D converter 30. The pulse-wave signal SM₁ represents a cuff pulse wave, i.e., a pressure wave which is produced from a brachial artery, not shown, of the patient in synchronism with the heartbeat of the patient and is propagated to the cuff 10.

The electronic control device 28 is provided by a so-called microcomputer including a CPU (central processing unit) 29, a ROM (read only memory) 31, a RAM (random access memory) 33 and an I/O (input-and-output) port, not shown. The CPU 29 processes signals according to the control programs pre-stored in the ROM 31 by utilizing the temporary-storage function of the RAM 33, and supplies drive signals to the switch valve 16 and the air pump 18 through the I/O port so as to perform a sequence of measuring actions in an oscillometric blood-pressure measuring operation and thereby measure a blood-pressure value of the patient. In addition, the CPU 29 operates a display device 32 to display the obtained blood-pressure value of the patient. The display device 32 may include a cathode ray tube (CRT).

The monitoring apparatus 8 further includes a photoelectric-pulse-wave detecting probe 38 (hereinafter, referred to as the "probe") which is employed as part of a pulse oximeter. The probe 38 functions as a pulse-wave detecting device for detecting a pulse wave that is propagated to a peripheral artery including capillaries. The probe 38 is adapted to be set on a body surface 40 of the subject, e.g., an end portion of a finger of the subject, with the help of a band, not shown, such that the probe 38 closely contacts the body surface 40. The probe 38 includes a container-like housing 42 which opens in one direction, a first and a second group of light emitting elements 44a, 44b, such as LEDs (light emitting diodes), which are disposed on an outer peripheral portion of an inner bottom surface of the housing 42 (hereinafter, referred to as the light emitting elements 44 in the case where the first and second group of light emitting elements 44a, 44b need not be discriminated from each other), a light receiving element 46, such as a photodiode or a phototransistor, which is disposed on a central portion of the inner bottom surface of the housing 42, a transparent resin 48 which is integrally disposed in the housing 42 to cover the light emitting elements 44 and the light receiving element 46, and an annular shade member 50 which is disposed between the light emitting elements 44 and the light receiving element 46, for preventing the lights emitted toward the body surface 40 by the light emitting elements 44 and then directly reflected from the body surface 40, from being received by the light receiving element 46.

The first and second groups of light emitting elements 44a, 44b emit, e.g., a red light having about 660 nm wavelength and an infrared light having about 800nm wavelength, respectively. The first and second light emitting elements 44a, 44b alternately emit the red and infrared lights at a predetermined frequency. The lights emitted toward the body surface 40 by the light emitting elements 44 are reflected from a body tissue of the subject where a dense capillaries occur, and the reflected lights are received by the common light receiving element 46. In place of the 660 nm and 800 nm wavelengths lights, the first and second light emitting elements 44a, 44b may emit various pairs of lights each pair of which have different wavelengths, so long as one light of the each pair exhibits significantly different absorption factors with respect to oxygenated hemoglobin and reduced hemoglobin, respectively, and the other light exhibits substantially same absorption factors with respect to the two sorts of hemoglobin.

The light receiving element 46 outputs, through a low-pass filter 52, a photoelectric-pulse-wave signal SM₃ representing the received or detected amount of light. The light receiving element 46 is connected to the low-pass filter 52 via an amplifier or the like. The low-pass filter 52 removes, from the photoelectric pulse-wave signal SM₃ input thereto, noise having frequencies higher than that of the pulse wave, and outputs the noise-free signal SM₃, to a demultiplexer 54. The photoelectric pulse wave represented by the photoelectric-pulse-wave signal SM₃ is a volumetric pulse wave which is produced in synchronism with the pulse of the patient.

The demultiplexer 54 is alternately switched according to signals supplied thereto from the control device 28 in synchronism with the light emissions of the first and second light emitting elements 44a, 44b. Thus, the demultiplexer 54 successively supplies, to the I/O port, not shown, of the control device 28, an electric signal SM_{R} representing the red light through a sample-and-hold circuit 56 and an A/D converter 58, and an electric signal SM_{IR} representing the infrared light through a sample-and-hold circuit 60 and an A/D converter 62. The sample-and-hold circuits 56, 60 hold the electric signals SM_{R}, SM_{IR} input thereto, respectively, and do not output those electric signals to the A/D converters 58, 62, before the prior signals SM_{R}, SM_{IR} are completely converted by the two A/D converters 58, 62, respectively.

In the control device 28, the CPU 29 generates a light emit signal SLV to a drive circuit 64 so that the first and second light emitting elements 44a, 44b alternately emit the red and infrared lights at a predetermined frequency, respectively, such that each light emission lasts for a predetermined duration. In synchronism with the alternate light emissions by the first and second light emitting elements 44a, 44b, the CPU 29 generates a switch signal SC to the demultiplexer 54 so as to correspondingly place the demultiplexer 54 in a first or a second position. Thus, the signals SM_{R}, SM_{IR} are separated from each other by the demultiplexer 54 such that the signal SM_{R} is supplied to the sample-and-hold circuit 56 while the signal SM_{IR} is supplied to the sample-and-hold circuit 60. Further, the CPU 29 periodically determines a degree of saturation of oxygen in blood of the subject, S_{PO2}, (%) based on respective amplitudes of the signals SM_{R}, SM_{IR}, according to a predetermined expression pre-stored in the ROM 31, and operates the display device 32 to display each of the determined degrees of blood oxygen saturation.

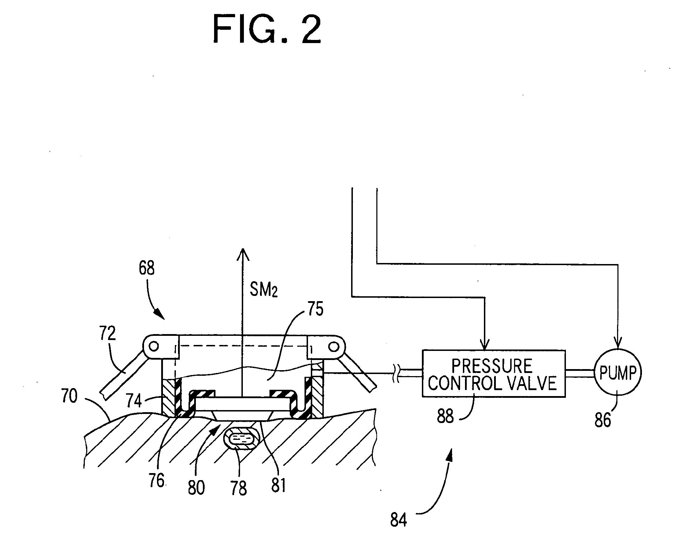

As shown in detail in Fig. 2, the pressure-pulse-wave sensor 68 includes a container-like housing 74 which is detachably attached, with fastening bands 72, to a body surface 70 of a body portion, such as a wrist, located on a downstream side of the artery of the upper arm 12 of the patient, such that an opening end of the housing 74 is opposed to the body surface 70. In addition, the sensor 68 includes a press member 80 which is secured via a diaphragm 76 to an inner wall of the housing 74, such that the press member 80 is movable relative to the housing 74 and is advanceable out of the opening of the same. The housing 74, the diaphragm 76, etc. cooperate with one another to define a pressure chamber 75, which is supplied with a pressurized air from an air pump 86 via a pressure control valve 88 so that the press member 80 is pressed against a radial artery 78 right below the body surface 70 with a pressing force P_{HD} corresponding to the air pressure in the pressure chamber 75.

The press member 80 includes a semiconductor chip that is provided by, e.g., a monocrystalline silicon and has a flat press surface 81, and a number of semiconductor pressure-sensing elements (not shown) that are arranged on the press surface 81 at regular intervals of, e.g., 0.2 mm in a direction perpendicular to the radial artery 78. The press member 80 is pressed against the radial artery 78 right below the body surface 70 of the wrist, to detect a pressure pulse wave, i.e., a pressure oscillation which is produced from the radial artery 78 and is transmitted to the body surface 70, and supplies a pressure-pulse-wave signal SM₂ representing the pressure pulse wave, to the electronic control device 28 via an A/D converter 82.

The CPU 29 of the control device 28 outputs drive signals to the air pump 86 and the pressure control valve 88 of a pressing-force changing device 84, and thereby controls the air pressure in the pressure chamber 175, i.e., the pressing force applied by the press member 80 to the skin, according to the control programs pre-stored in the ROM 31. In a continuous blood-pressure measuring operation, the control device 28 determines, based on the pressure pulse wave signal SM₂ continuously detected while the pressure in the pressure chamber 75 is changed, an optimum pressing pressure P_{HDP} at which the press member 80 is pressed against the radial artery 78 such that a portion of the wall of the artery 78 is flattened. The control device 28 controls the pressure control valve 88 so as to maintain the pressure of the pressure chamber 75 at the thus determined optimum pressing pressure P_{HDP}.

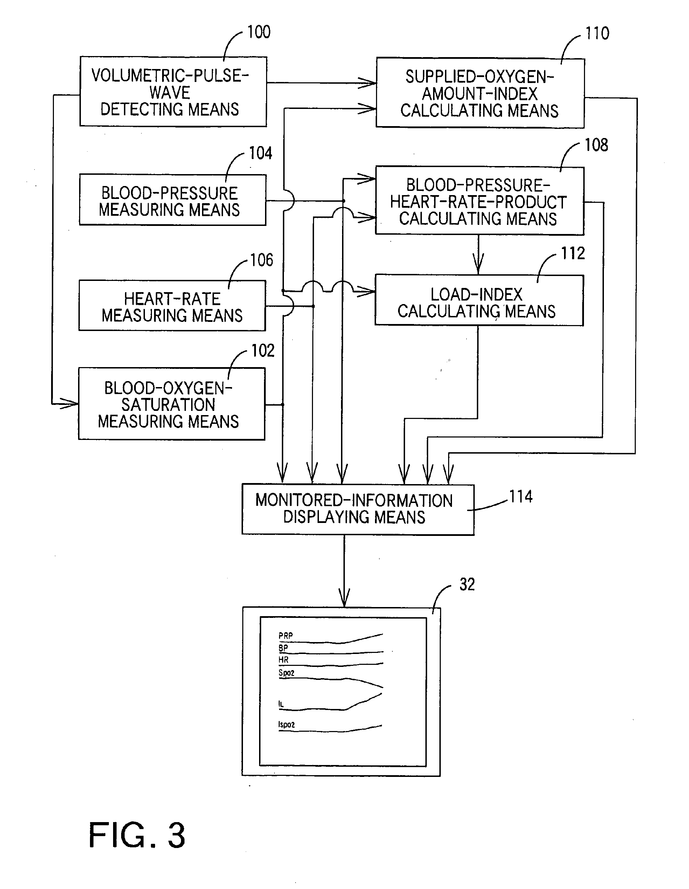

Fig. 3 is a diagrammatic view for explaining essential control functions of the electronic control device 28 of the living-subject monitoring apparatus. In the figure, a volumetric-pulse-wave detecting device or means 100 continuously obtains, from the low-pass filter 52, the photoelectric-pulse-wave signal SM₃ having a magnitude corresponding an amount of the light received by the light receiving element 46, and thereby detects a plurality of successive heartbeat-synchronous pulses of the volumetric pulse wave that are produced in synchronism with respective heartbeats of the subject. A blood-oxygen-saturation measuring device or means 102 iteratively determines, at a predetermined period, a blood oxygen saturation S_{PO2} (%) of the subject based on respective amplitudes of the electric signals SM_{R}, SM_{IR} obtained from the light receiving element 46 by producing the switch signals SC in synchronism with the respective light emissions of the light emitters 44a, 44b caused by the drive circuits 64 and thereby switching the demultiplexer 54, according to the expression pre-stored in the ROM 31 for determination of blood-oxygen saturation.

A blood-pressure measuring device or means 104 successively determines an estimated blood pressure EBP of the subject based on a magnitude of each of successive heartbeat synchronous pulses of the pressure-pulse-wave signal SM₂ obtained from the pressure-pulse-wave sensor 68, according to a relationship that is determined, at a prescribed calibration period, based on blood pressure values measured using the cuff 10 and magnitudes of the pressure-pulse-wave signal SM₂. A heart-rate measuring device or means 106 determines a heart rate HR (= number of heartbeats / unit time) of the subject based on the pressure-pulse-wave signal SM₂ or the photoelectric-pulse-wave signal SM₃.

A blood-pressure-heart-rate-product calculating device or means 108 iteratively calculates a product PRP of a blood pressure BP iteratively measured by the blood-pressure measuring means 104 and a heart rate HR iteratively measured by the heart-rate measuring means 106. Each of the blood pressure BP, the heart rate HR, and the blood pressure-heart rate product PRP is a circulatory-organ-related parameter that changes in relation with a load exerted to the subject; and each of the blood-pressure measuring means 104, the heart-rate measuring means 106, and the blood-pressure-heart-rate-product calculating means 108 is a circulatory-organ-related parameter measuring device or means.

A supplied-oxygen-amount-index calculating device or means 110 first calculates an area S defined by each of heartbeat- synchronous pulses of the photoelectric-pulse-wave signal SM₃, i.e., the volumetric pulse wave detected by the volumetric-pulse- wave detecting means 100, and then calculates a product of the thus calculated area S and an oxygen saturation S_{PO2} iteratively measured by the blood-oxygen-saturation measuring means 102, thereby calculating a supplied-oxygen-amount index I_{SPO2} indicating an amount of oxygen supplied to the subject. The area S of each of heartbeat-synchronous pulses of the photoelectric-pulse-wave signal SM₃ may be replaced with an amplitude of the each heartbeat-synchronous pulse of the signal SM₃ that can be regarded as substantially corresponding to the area.

A load-index calculating device or means 112 calculates a load index I_{L} indicating a load exerted to the subject, such that the load index is equal to a value (=PRP/S_{PO2}) obtained by dividing the circulatory-organ-related parameter, such as the blood pressure BP, the heart rate HR, or the blood pressure-heart rate product PRP, by an oxygen saturation S_{PO2} iteratively measured by the blood-oxygen-saturation measuring means 102. Thus, the magnitude of the load exerted to the subject can be easily recognized.

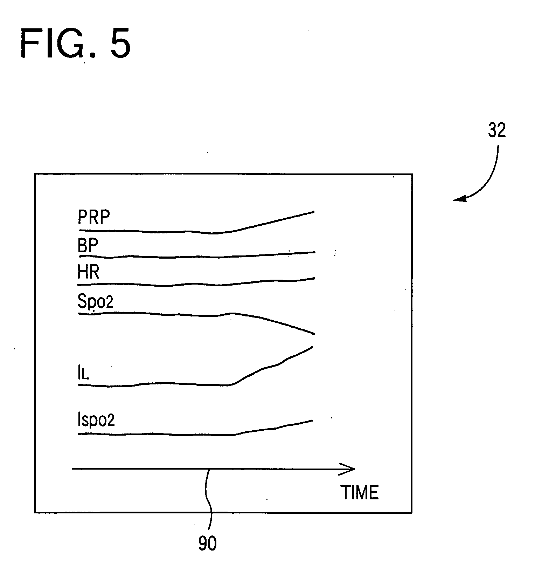

A monitored-information displaying device or means 114 operates the display device 32 to display, in a screen image thereof shown in Fig. 5, respective time-wise trends of the estimated blood pressure EBP, the heart rate HR, the blood pressure-heart rate product PRP, the oxygen saturation S_{PO2}, the supplied-oxygen-amount index I_{SPO2}, and the load index I_{L}, in a two-dimensional graph having a common time axis 90.

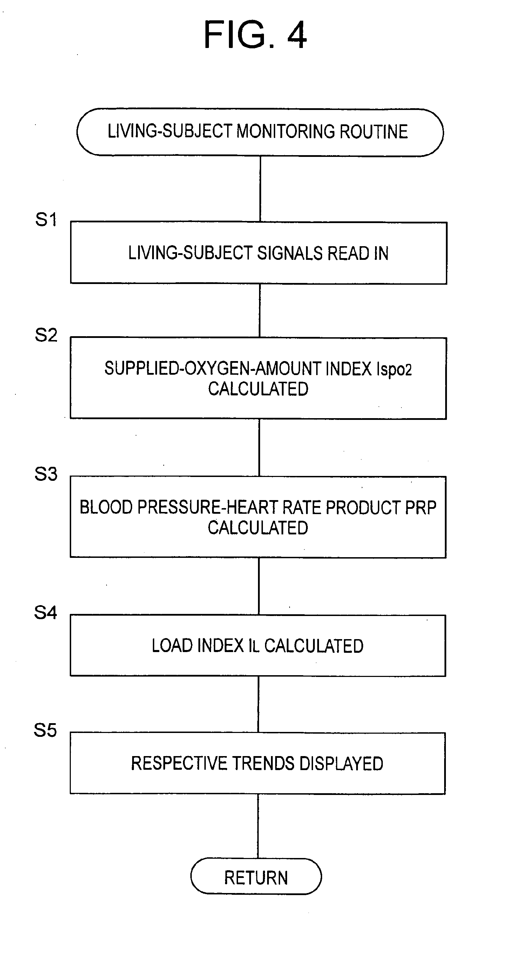

Fig. 4 is a flow chart for explaining the essential control functions of the electronic control device 28. At Step S1 (hereinafter, "Step" is omitted) of Fig. 4, the control device reads in living-subject signals including the photoelectric-pulse-wave signal SM₃, the electric signals SM_{R}, SM_{IR}, and the pressure-pulse-wave signal SM₂. In addition, the control device reads in living-subject information including a blood pressure BP, a heart rate HR, and an oxygen saturation S_{PO2} that are calculated from those signals. Subsequently, the control proceeds with S2 corresponding to the supplied-oxygen-amount-index calculating means 110. At S2, the control device calculates an area S defined by a heartbeat-synchronous pulse of the volumetric pulse wave represented by the photoelectric-pulse-wave signal SM₃, and calculates, as a supplied-oxygen-amount index I_{SPO2} indicating an amount of oxygen supplied to the subject, the product of the area S and an oxygen saturation S_{PO2} that is iteratively measured. Next, the control goes to S3 corresponding to the blood-pressure-heart-rate-product calculating means 108, the control device calculates a product PRP of an estimated blood pressure EBP and a heart rate HR that are iteratively measured. Subsequently, the control goes to S4 corresponding to the load-index calculating means 112. At S4, the control device calculates a load index I_{L} indicating a load exerted to the subject, such that the load index is equal to a value (=EBP/S_{PO2}, HR/S_{PO2}, PRP/S_{PO2}) obtained by dividing a circulatory-organ-related parameter, such as a blood pressure BP, a heart rate HR, or a blood pressure-heart rate product PRP, by an oxygen saturation S_{PO2} iteratively measured by the blood-oxygen-saturation measuring means 102. Thus, a change of the magnitude of the load exerted to the subject can be easily recognized. Then, the control goes to S5 corresponding to the monitored-information displaying means 114. At S5, the control device operates the display device 32 to display, in the screen image thereof shown in Fig. 5, respective time-wise trends of the estimated blood pressure EBP, the heart rate HR, the blood pressure-heart rate product PRP, the oxygen saturation S_{PO2}, the supplied-oxygen-amount index I_{SPO2}, and the load index I_{L}, in the two-dimensional graph having the common time axis 90.

It emerges from the foregoing description of the present embodiment that the monitored-information displaying means 114 (S5) operates the display device to display the circulatory-organ-related parameter (i.e., the estimated blood pressure EBP, the heart rate HR, and the blood pressure-heart rate product PRP) that changes in relation with the load exerted to the subject, and the blood oxygen saturation S_{PO2} that represents the degree of saturation of oxygen in blood of the subject, such that the circulatory-organ-related parameter and the blood oxygen saturation are comparable with each other by a living person such as a medical person or the subject. Therefore, based on an increasing tendency of the circulatory-organ-related parameter and a decreasing tendency of the blood oxygen saturation, the person can quickly recognize that the ventilating ability of lungs of the subject whose circulatory organ is undergoing the load is abnormally or insufficiently low.

Also, according to this embodiment, the circulatory-organ-related-parameter measuring means (i.e., the blood-pressure measuring means 104, the heart-rate measuring means 106, and the blood-pressure-heart-rate-product calculating means 108) measures the circulatory-organ-related parameter, i.e., the estimated blood pressure EBP, the heart rate HR, and the blood pressure-heart rate product PRP. Therefore, based on respective increasing tendencies of the estimated blood pressure EBP, the heart rate HR, and the blood pressure-heart rate product PRP and a decreasing tendency of the blood oxygen saturation S_{PO2}, it is possible to quickly recognize that the ventilating ability of lungs of the subject whose circulatory organ is undergoing the load is abnormally or insufficiently low.

Also, according to this embodiment, the monitored-information displaying means 114 (S5) operates the display device 32 to display the circulatory-organ-related parameter (i.e., the estimated blood pressure EBP, the heart rate HR, and the blood pressure-heart rate product PRP), and the blood oxygen saturation S_{PO2} that represents the degree of saturation of oxygen in blood of the subject, such that respective time-wise trends of the circulatory-organ-related parameter and the blood oxygen saturation are comparable with each other by a living person. Thus, an increasing tendency of the circulatory-organ-related parameter (i.e., the estimated blood pressure EBP, the heart rate HR, and the blood pressure-heart rate product PRP), and a decreasing tendency of the blood oxygen saturation S_{PO2}, can be easily recognized.

Also, according to the present embodiment, the supplied-oxygen-amount-index calculating means 110 (S2) iteratively calculates the area S of each heartbeat-synchronous pulse of the volumetric pulse wave represented by the photoelectric-pulse-wave signal SM₃, and iteratively calculates, as the supplied-oxygen-amount index I_{SPO2} indicating the amount of oxygen supplied to the subject, the product of the area S and the oxygen saturation S_{PO2} that is iteratively measured. Thus, based on the supplied-oxygen-amount index I_{SPO2}, it is possible to quickly recognize or detect that the ventilating ability of lungs of the subject whose circulatory organ is undergoing the load is abnormally or insufficiently low.

Also, according to this embodiment, the circulatory-organ-related-parameter measuring means (i.e., the blood-pressure measuring means 104, the heart-rate measuring means 106, and the blood-pressure-heart-rate-product calculating means 108) measures the circulatory-organ-related parameter (i.e., the estimated blood pressure EBP, the heart rate HR, and the blood pressure-heart rate product PRP) that changes in relation with the load exerted to the subject, and the supplied-oxygen-amount-index calculating means 110 calculates the supplied-oxygen-amount index I_{SPO2}; and the monitored-information displaying means 114 (S5) operates the display device to display the circulatory-organ-related parameter and the supplied-oxygen-amount index I_{SPO2}, such that respective time-wise trends of the circulatory-organ-related parameter and the supplied-oxygen-amount index are comparable with each other by a living person. Therefore, based on an increasing tendency of the circulatory-organ-related parameter and a decreasing tendency of the supplied-oxygen-amount index, it is possible to quickly recognize that the ventilating ability of lungs of the subject whose circulatory organ is undergoing the load is abnormally or insufficiently low.

Also, according to this embodiment, the load-index calculating means 112 (S4) iteratively calculates, based on the value obtained by dividing the circulatory-organ-related parameter (i.e., the estimated blood pressure EBP, the heart rate HR, and the blood pressure-heart rate product PRP) iteratively measured by the blood-oxygen-saturation measuring means (i.e., the blood-pressure measuring means 104, the heart-rate measuring means 106, and the blood-pressure-heart-rate-product calculating means 108), by the blood oxygen saturation S_{PO2} measured by the blood-oxygen-saturation measuring means 102, the load index I_{L} indicative of the load exerted to the subject. Based on the load index I_{L}, it is possible to quickly recognize or detect that the ventilating ability of lungs of the subject whose circulatory organ is undergoing the load is abnormally or insufficiently low. Particularly, in the case where the load-index calculating means calculates, based on the value obtained by dividing the blood pressure-heart rate product PRP by the blood oxygen saturation S_{PO}2 measured by the blood-oxygen-saturation measuring means 102, the load index indicative of the load exerted to the subject. Thus, based on the load index I_{L} that reflects the respective changes of the load and the oxygen saturation, it is possible to more quickly recognize that the ventilating ability of lungs of the subject whose circulatory organ is undergoing the load is abnormally or insufficiently low.

Also, according to this embodiment, the monitored-information displaying means 114 (S5) operates the display device to display the load index I_{L} calculated by the load-index calculating means 112 (S4) and the circulatory-organ-related parameter (i.e., the estimated blood pressure EBP, the heart rate HR, and the blood pressure-heart rate product PRP) measured by the blood-oxygen-saturation measuring means (i.e., the blood-pressure measuring means 104, the heart-rate measuring means 106, and the blood-pressure-heart-rate-product calculating means 108), such that respective time-wise trends of the load index and the circulatory-organ-related parameter are comparable with each other by a living person. Thus, a time-wise relative change between an increasing tendency of the circulatory-organ-related parameter, such as the estimated blood pressure EBP, the heart rate HR, and the blood pressure-heart rate product PRP, and an increasing tendency of the load index I_{L}, can be easily recognized.

While the present invention has been described in its preferred embodiment by reference to the drawings, it is to be understood that the invention may otherwise be embodied.

For example, in the illustrated embodiment, the blood-pressure measuring means 104 successively determines an estimated blood pressure EBP of the subject based on each of successive heartbeat-synchronous pulses of the pressure pulse wave. However, the blood-pressure measuring means 104 may be replaced with a blood-pressure measuring device that measures, using the cuff 10, a blood pressure of the subject at regular intervals of from several minutes to several tens of minutes according to oscillometric method or Korotkoff-sound method.

Also, in the illustrated embodiment, each of the estimated blood pressure EBP, the pulse rate HR, and the blood pressure-heart rate product PRP is measured as the circulatory-organ-related parameter. However, appropriate coefficient and/or constant may be added to the each parameter.

Also, in the illustrated embodiment, a supplied-oxygen-amount index I_{SPO2} is iteratively calculated as the product of area S or amplitude of each of successive heartbeat-synchronous pulses of the photoelectric-pulse-wave signal SM₃ continuously obtained and an oxygen saturation S_{PO2} successively measured. However, appropriate coefficient and/or constant may be added to the expression used for calculating index I_{SPO2}, or each index value I_{SPO2} calculated. That is, the index I_{SPO2} may be replaced with any parameter which is obtained based on the product of area S or amplitude of the photoelectric-pulse-wave signal SM₃ reflecting an amount of blood supplied to the subject, and the blood oxygen saturation S_{PO2} reflecting an amount of oxygen in blood of the subject, and which accordingly reflects an amount of oxygen supplied to the subject.

Also, in the illustrated embodiment, the load index I_{L} is calculated as the value obtained by dividing the circulatory-organ-related parameter (i.e., the estimated blood pressure EBP, the heart rate HR, or the blood pressure-heart rate product PRP) by the oxygen saturation S_{PO2}. However, appropriate coefficient and/or constant may be added to the expression used for calculating the index I_{L}, or each index value I_{L} calculated. That is, the index I_{L} may be replaced with any parameter which is obtained by dividing the circulatory-organ-related parameter that increases with the load exerted to the subject, by the oxygen saturation S_{PO2} that decreases with the increasing of the load, and which accordingly emphasizes the change (i.e., increase) of the load exerted to the subject.

In addition, in the illustrated embodiment, each of the volumetric-pulse-wave detecting means 100, the blood-pressure measuring means 104, the pulse-rate measuring means 106, the blood-oxygen-saturation measuring means 102, the supplied-oxygen-amount-index calculating means 110, the blood-pressure-heart-rate-product calculating means 108, and the load-index calculating means 112 may be provided outside the casing in which the display device 32 is provided, and may be connected to the display device 32 either on an on-line basis using a wire or a wireless line, or on an off-line basis.

In addition, in the illustrated embodiment, the photoelectric-pulse-wave detecting probe 38 is of reflection type. However, the reflection-type probe 38 may be replaced with a transmission-type probe that is adapted to be worn on, e.g., an earlobe or a finger.

In addition, in the illustrated embodiment, the display device 32 of the living-subject monitoring apparatus 8 displays not only the respective time-wise trends of the estimated blood pressure EBP, the pulse rate HR, and the blood pressure-heart rate product PRP each as the circulatory-organ-related parameter, but also the supplied-oxygen-amount index I_{SPO2} and the load index I_{L}. However, the display device 32 may be modified to display only a portion of those parameters. In addition, the display device 32 may be modified to display those parameters not in the form of respective time-wise trends thereof shown in Fig. 5, but in the form of respective digits or graphs.

It is to be understood that the present invention may be embodied with other changes, improvements, and modifications that may occur to a person skilled in the art without departing from the spirit and scope of the invention defined in the appended claims.

## Claims

1. A living-subject monitoring apparatus (8) for monitoring a load exerted to a living subject, comprising:
a circulatory-organ-related-parameter measuring device (10, 14, 22, 24, 80, 104, 106, 108) which iteratively measures at least one circulatory-organ-related parameter of the subject that changes in relation with the load exerted to the subject;
a blood-oxygen-saturation measuring device (38, 100, 102) which iteratively measures a blood oxygen saturation of the subject; and
a display device (32, 114) which simultaneously displays the circulatory-organ-related parameter measured by the circulatory-organ-related-parameter measuring device and the blood oxygen saturation measured by the blood-oxygen-saturation measuring device, so that the circulatory-organ-related parameter and the blood oxygen saturation are comparable with each other.

2. A living-subject monitoring apparatus according to claim 1, wherein said at least one circulatory-organ-related parameter measured by the circulatory-organ-related-parameter measuring device (10, 14, 22, 24, 80, 104, 106, 108) comprises at least one of a blood pressure, a heart rate, and a product of a blood pressure and a heart rate, of the subject.

3. A living-subject monitoring apparatus according to claim 1 or claim 2, wherein the display device (32, 114) simultaneously displays respective values of the circulatory-organ-related parameter that are iteratively measured by the circulatory-organ-related-parameter measuring device, and respective values of the blood oxygen saturation that are iteratively measured by the blood-oxygen-saturation measuring device, so that the respective values of the circulatory-organ-related parameter and the respective values of the blood oxygen saturation are comparable with each other.

4. A living-subject monitoring apparatus (8) for monitoring a load exerted to a living subject, comprising:
a blood-oxygen-saturation measuring device (38, 100, 102) which iteratively measures a blood oxygen saturation of the subject;
a volumetric-pulse-wave detecting device (38, 100) which iteratively detects a volumetric pulse wave from the subject; and
a supplied-oxygen-amount-index calculating device (110) which iteratively calculates, based on a product of the blood oxygen saturation measured by the blood-oxygen-saturation measuring device and a volumetric-pulse-wave-related parameter related to the volumetric pulse wave detected by the volumetric-pulse-wave detecting device, a supplied-oxygen-amount index indicative of an amount of oxygen supplied to the subject.

5. A living-subject monitoring apparatus (8) according to claim 4, further comprising:
a circulatory-organ-related-parameter measuring device (10, 14, 22, 24, 80, 104, 106, 108) which iteratively measures at least one circulatory-organ-related parameter of the subject that changes in relation with the load exerted to the subject; and
a display device (32, 114) which simultaneously displays respective values of the circulatory-organ-related parameter that are iteratively measured by the circulatory-organ-related-parameter measuring device, and respective values of the supplied-oxygen-amount index that are iteratively calculated by the supplied-oxygen-amount-index calculating device, so that the respective values of the circulatory-organ-related parameter and the respective values of the supplied-oxygen-amount index are comparable with each other.

6. A living-subject monitoring apparatus (8) for monitoring a load exerted to a living subject, comprising:
a circulatory-organ-related-parameter measuring device (10, 14, 22, 24, 80, 104, 106, 108) which iteratively measures at least one circulatory-organ-related parameter of the subject that changes in relation with the load exerted to the subject;
a blood-oxygen-saturation measuring device (38, 100, 102) which iteratively measures a blood oxygen saturation of the subject; and
a load-index calculating device (112) which iteratively calculates, based on a value obtained by dividing the circulatory-organ-related parameter measured by the blood-oxygen-saturation measuring device, by the blood oxygen saturation measured by the blood-oxygen-saturation measuring device, a load index indicative of the load exerted to the subject.

7. A living-subject monitoring apparatus according to claim 6, further comprising a display device (32, 114) which simultaneously displays respective values of the circulatory-organ-related parameter measured by the circulatory-organ-related-parameter measuring device, and respective values of the load index calculated by the load-index calculating device, so that the respective values of the circulatory-organ-related parameter and the respective values of the load index are comparable with each other.

8. A living-subject monitoring apparatus according to claim 6 or claim 7, wherein said at least one circulatory-organ-related parameter measured by the circulatory-organ-related-parameter measuring device (10, 14, 22, 24, 80, 104, 106, 108) comprises a product of blood pressure and heart rate of the subject, and wherein the load-index calculating device (112) calculates, based on a value obtained by dividing the product of blood pressure and heart rate measured by the circulatory-organ-related-parameter measuring device, by the blood oxygen saturation measured by the blood-oxygen-saturation measuring device, the load index indicative of the load exerted to the subject.

9. A living-subject monitoring apparatus according to claim 4 or claim 5, wherein the volumetric-pulse-wave-related parameter comprises an area defined by a waveform of each of a plurality of heartbeat-synchronous pulses of the volumetric pulse wave detected by the volumetric-pulse-wave detecting device.

10. A living-subject monitoring apparatus according to claim 4 or claim 5, wherein the volumetric-pulse-wave-related parameter comprises an amplitude of a waveform of each of a plurality of heartbeat-synchronous pulses of the volumetric pulse wave detected by the volumetric-pulse-wave detecting device.
